# EUROPÄISCHE PATENTSCHRIFT

(11) **EP 1 161 241 B1**
(45) Veröffentlichungstag und Bekanntmachung des Hinweises auf die Patenterteilung: **07.12.2005**
(21) Anmeldenummer: 00916920.2
(22) Anmeldetag: 10.03.2000
(51) Int. Cl.: A61K 31/4525, A61K 47/44, A61K 9/48

(54) **STABILE PHARMAZEUTISCHE ANWENDUNGSFORM FÜR PAROXETIN-ANHYDRAT**
STABLE PHARMACEUTICAL APPLICATION FORM FOR PAROXETIN ANHYDRATE
FORME GALENIQUE PHARMACEUTIQUE STABLE POUR ANHYDRATE DE PAROXETINE

(30) Priorität: 12.03.1999 DE 19910954
(43) Veröffentlichungstag der Anmeldung: 12.12.2001
(73) Patentinhaber: Aesica Pharmaceuticals Ltd., Cramlington Northumberland, NE23 3JL (GB)
(72) Erfinder: FISCHER, Gerhard, D-69412 Eberbach (DE); EINIG, Heinz, D-67433 Neustadt (DE); REIDELSHÖFER, Alfred, D-64720 Michelstadt (DE)
(74) Vertreter: Hayes, Adrian Chetwynd
(86) Internationale Anmeldenummer: PCT/EP2000/002120
(87) Internationale Veröffentlichungsnummer: WO 2000/054775

(56) Entgegenhaltungen:
- WO-A-98/29136
- WO-A-99/00131
- WO-A-99/26625
- WO-A-99/58116

## Beschreibung

Paroxetin, (-) trans-4-(4'-Fluorphenyl)-3-(3',4'-methylendioxy-phenoxymethyl)-piperidin ist in den US-Patenten US-A-3,912,743 und US-A-4,007,196 beschrieben und wird als Antidepressivum eingesetzt. Die übliche Verabreichungsform stellt dabei das Hydrochlorid dar. Für den allgemeinen pharmazeutischen Gebrauch und die Formulierung von Arzneimitteln ist das Hemihydrat (½ H₂O) die übliche Form.

Die EP-B-0 223 403 beschreibt sowohl das Anhydrat als auch das Hemihydrat. Bekannt sind auch organische Solvate, insbesondere mit 2-Propanol, wie dies in der WO-A-96/24595 beschrieben ist.

Die oben erwähnte EP-B-0 223 403 beschreibt die Herstellung des Anhydrats durch scharfe Trocknung des Hemihydrates, wobei jedoch die Rehydratation zum Hemihydrat sehr schnell erfolgt.

WO-A-98/31365 beschreibt die Herstellung einer "free-flowing and easily soluble form" als Hemihydrat oder Solvat. Die Herstellung eines geschmacksgeschützten Paroxetin gemäß der WO-A-95/15155 beruht auf dem Einsatz eines Komplexes der freien Basen mit einem Copolymer aus Methacrylsäure und Methylmethacrylsäure.

Die Herstellung einer slow-release Form wird in der WO-A-96/31197 beschrieben, in der auf den Einsatz von Paroxetinhydrochlorid verwiesen wird. Es ist kein konkretes Beispiel für Paroxetin angegeben, aufgrund der zur Retardierung eingesetzten Hilfsstoffe, die bereits einen größeren Gehalt an Wasser besitzen, und des verwendeten Schmelzprozesses kann gemäß dem dort beschriebenen Verfahren auch beim Einsatz des Paroxetinhydrochlorid-Anhydrats nur das Hemihydrat oder ein höheres Hydrat gebildet werden.

Kombinationen von 5-Hydroxytryptamin. (5HT) Uptake Inhibitors (unter anderem von Paroxetin) mit weiteren Wirkstoffen zur Erzielung spezieller pharmakologischer und therapeutischer. Effekte sind beschrieben in der WO-A-96/41633, GB-A-2 303 303, WO-A-97/31629, US-A-5,776,969, WO-A-98/44924, EP-A-0 714 663 und der WO-A-98/11897. Die angegebenen Anwendungsbeispiele und die beschriebenen, nicht wasserfreien Präparationen deuten auf den Einsatz bzw. die anschließende Bildung des Paroxetin Hemihydrates hin.

WO-A-97/03670 beschreibt eine slow-release Form von Paroxetin, wobei ausdrücklich der Einsatz der freien Base oder des Hemihydrates erwähnt ist.

Aus der US-A-5,872,132 sind vier verschiedene Formen des Paroxetinhydrochlorid-Anhydrats bekannt, von denen nur die Form C beansprucht wird. Weiterhin wird in Spalte 7, Zellen 9 - 39 in allgemeiner Form beschrieben, wie aus dieser Form orale Applikationsformen hergestellt werden könnten.

In der nicht vorpublizierten WO-A-99/00131 sind Weichgelatinekapseln beschrieben, enthaltend Paroxetin als wasserlösliche, feste Dispersion.

Es sind jedoch bisher keine Arzneimittel bekannt, die Paroxetin oder seine pharmazeutisch verträglichen Salze als Anhydrat enthalten. Dies ist wohl darauf zurückzuführen, dass diese Formen des Anhydrats bereits bei der Konfektionierung Wasser aufnehmen und wieder das Hemihalbhydrat bilden.

Überraschenderweise wurde nun gefunden, daß es möglich ist, Paroxetin-Anhydrat oder ein pharmazeutisch verträgliches Salz in einer stabilen Form ohne Umwandlung in das Hemihydrat in ein Arzneimittel zu überführen, wenn der Wirkstoff zusammen mit lipophilen Trägerstoffen, nämlich Silikonölen, eingesetzt wird. Als verträgliche Salze sind z.B. das Sulfat, Methylsulfat, Phosphat oder Carbonat einsetzbar. Das bevorzugt eingesetzte pharmazeutisch verträgliche Salz ist das Paroxetinhydrochlorid-Anhydrat.

Vorzugsweise sind die Trägerstoffe bei Raumtemperatur halbfest oder flüssig.

Das erfindungsgemäße Arzneimittel liegt in Form von Weichgelatinekapseln vor.

Die Einbringung des Paroxetin-Anhydrats in Weichgelatinekapseln war überraschend, da die Weichgelatine beim Verkapselungsprozeß sehr große Mengen an Wasser enthält. Es wurde jedoch experimentell überprüft, daß keine Umwandlung zum Hemihydrat stattgefunden hat. Es sollte jedoch vermieden werden, Glycerin bei der Herstellung der Kapselhülle zu verwenden.

Eine Prüfung auf Abwesenheit des Hemihydrats kann sehr einfach durch Infrarot-Spektroskopie oder Differential Scanning Calorimetrie (DSC) erfolgen, wie dies von P.C. Baxter et al. im International Journal of Pharmaceutics, 42 (1988) Seiten 135 bis 143 beschrieben ist.

Auch das Verfahren zur Herstellung des erfindungsgemaßen Arzneimittels, bei dem Paroxetin-Anhydrat oder ein pharmazeutisch verträgliches Salz davon in lipophilen Trägerstoffen, nämlich Silikonölen, suspendiert und die Suspension in Weichgelatinekapseln abgefüllt wird, ist Bestandteil der Erfindung.

Das folgende Beispiel soll die Erfindung näher erläutern.

### Weichgelatinekapseln

Es wird in der Kälte eine Mischung aus

| | |
|---|---|
| Siliconöl, niedrig viskos | 99,7% |
| Siliciumdioxid, hochdispers | 0,3% |

hergestellt. Pro 207,2 mg Gesamtmischung werden 22,8 mg Paroxetinhydrochlorid-Anhydrat zugegeben. Die fertige Mischung wird dann in bekannter Weise zu Weichgelatinekapseln weiterverarbeitet. Zweckmäßigerweise wird der Kapselhülle kein Glycerol zugesetzt.

## Patentansprüche

1. Arzneimittel, enthaltend Paroxetin-Anhydrat oder pharmazeutisch verträgliche Salze davon zusammen mit lipophilen Trägerstoffen in Form einer Weichgelatinekapsel, wobei die lipophilen Trägerstoffe Silikonöle sind.

2. Arzneimittel nach Anspruch 1, **dadurch gekennzeichnet, dass** das pharmazeutisch verträgliche Salz Paroxetinhydrochlorid-Anhydrat ist.

3. Arzneimittel nach Ansprüchen 1 oder 2, **dadurch gekennzeichnet, dass** die Trägerstoffe bei Raumtemperatur halbfest oder flüssig sind.

4. Verfahren zur Herstellung eines Arzneimittels nach einem der Ansprüche 1 bis 3, **dadurch gekennzeichnet, dass** Paroxetin-Anhydrat oder ein pharmazeutisch verträgliches Salz in lipophilen Trägerstoffen, nämlich Silikonölen, suspendiert und die Suspension in Weichgelatinekapseln abgefüllt wird.

5. Verfahren gemäß Anspruch 4, **dadurch gekennzeichnet, dass** dem Silikonöl hochdisperses Siliciumdioxid im Verhältnis 99,7 : 0,3 zugemischt wird.

## Claims

1. A medicament containing paroxetin anhydrate or pharmaceutically acceptable salts together with lipophilic carriers in the form of a soft gelatin capsule, wherein the lipophilic carriers are silicone oils.

2. A medicament according to Claim 1, **characterised in that** the pharmaceutically acceptable salt is paroxetin hydrochloride anhydrate.

3. A medicament according to Claim 1 or 2, **characterised in that** the carriers are semisolid or liquid at room temperature.

4. A method for the preparation of a medicament according to any one of Claims 1 to 3, **characterised in that** paroxetin anhydrate or a pharmaceutically acceptable salt is suspended in lipophilic carriers, namely silicone oils, and the suspension is filled into soft gelatin capsules.

5. A method according to claim 4, **characterised in that** highly disperse silica is admixed with the silicone oil in a ratio of 99.7:0.3.

## Revendications

1. Médicament contenant de l'anhydrate de paroxétine ou des sels acceptables du point de vue pharmaceutique de ce composé en association avec des supports lipophiles sous la forme d'une capsule molle de gélatine, dans lequel les supports lipophiles sont des huiles de silicone.

2. Médicament selon la revendication 1, **caractérisé en ce que** le sel acceptable du point de vue pharmaceutique est l'anhydrate du chlorhydrate de paroxétine.

3. Médicament selon la revendication 1 ou 2, **caractérisé en ce que** les supports sont semi-solides ou liquides à la température ambiante.

4. Procédé de préparation d'un médicament selon l'une des revendications 1 à 3, **caractérisé en ce que** de l'anhydrate de paroxétine ou un sel acceptable du point de vue pharmaceutique est mis en suspension dans des supports lipophiles, à savoir des huiles de silicone, et la suspension est chargée dans des capsules molles de gélatine.

5. Procédé selon la revendication 4, **caractérisé en ce que** l'huile de silicone est additionnée de dioxyde de silicium hautement dispersé dans la proportion de 99,7 : 0,3.
